# EUROPÄISCHE PATENTANMELDUNG

(11) **EP 0 865 769 A1**
(43) Veröffentlichungstag der Anmeldung: **23.09.1998**
(21) Anmeldenummer: 97810164.0
(22) Anmeldetag: 19.03.1997
(51) Int. Cl.: A61B 17/72

(54) **Modularer Marknagel**

(71) Anmelder: OSTEO AG, 2545 Selzach (CH)
(72) Erfinder: Wahl, Thomas, 2543 Lengnau (CH); Bühren, Volker, 82418 Murnau (DE)
(74) Vertreter: Liebetanz, Michael, Dipl.-Phys.

(57) **Zusammenfassung**

Der modulare, unaufgebohrte Femur-Marknagel weist einen Nagel (1) auf, der aus einem proximalen Teil (2), Mittel-Teil (3) und distalen Teil (4) besteht, wobei das proximale Teil (2) einen Längsschlitz (8) sowie darunter angeordnet mindestens eine Querbohrung (10) und das distale Teil (4) mindestens eine Querbohrung (15) aufweisen. Das proximale Teil (2) weist eine sich über die Länge des Längsschlitzes (8) hinziehende Längsbohrung (5) zur Aufnahme eines Einsatzes (7) auf. Der Einsatz (7) ist mit zwei geneigt bezüglich der Längsachse verlaufenden Führungsbohrungen (29) zur Aufnahme von Verriegelungs-Schrauben (18), -Bolzen (19) oder Klingen versehen.

Ein solcher, vielseitig einsetzbarer modularer Marknagel weist eine hohe biomechanische Stabilität auf, wobei das proximale Ende des Stabes nicht zusätzlich verdickt ist.

## Beschreibung

Die vorliegende Erfindung bezieht sich auf einen modularen Marknagel, insbesondere auf einen modularen Femur-Marknagel gemäss Oberbegriff von Patentanspruch 1.

Seit einiger Zeit werden sogenannte unaufgebohrte Verriegelungsmarknägel zur Frakturbehandlung der Rohrenknochen eingesetzt. In letzter Zeit wurden die Indikationsstellungen dieser Marknägel auf die proximalen Femurschaft-Frakturen erweitert, wobei diese Frakturen in einer grossen Vielfalt vorliegen und die Behandlung dieser Frakturen äusserst komplex ist. Dies hat zur Folge, dass eine Vielzahl von Marknägeln entwickelt wurden, die dem jeweiligen Frakturtyp Rechnung trägt. Dabei unterscheiden sich diese Nägel hauptsächlich nur in der Art, wie die proximalen Verriegelungsschrauben gemäss Frakturtyp zu liegen kommen. Dadurch entsteht eine umfangreiche, kostenaufwendige Lagerhaltung der Nägel und der Instrumentarien. Ausserdem wird es für den Chirurgen und für die Operationsschwestern immer komplizierter, eine Vielzahl diverser Implantatsysteme in der Art zu beherrschen, dass eine optimale Frakturbehandlung gewährleistet ist.

Die WO-94/13290 beschreibt einen modular aufgebauten, unaufgebohrten Marknagel, der die Anzahl bereitzustellender Marknägel erheblich reduziert, indem er vielfältig eingesetzt werden kann. Dabei wird der Marknagel mit einer auf das proximale Ende teleskopisch aufschiebbaren, zylindrischen Hülse versehen, die durch ihre Ausbildung vielseitig angebracht werden kann, um den Marknagel an die jeweilige Verwendung anpassen zu können. Ein solcher Teleskopaufsatz ist jedoch biomechanisch nicht optimal, was aufgrund der grossen Belastungen am proximalen Femur als wesentlich zu betrachten ist, und des weiteren wird durch die aufgesetzte Hülse der proximale Nagelteil zusätzlich verdickt, was im Bezug auf die Vaskularisierung des Femurkopf-Halsbereiches negative Auswirkungen hat. Schliesslich verlängert der Teleskopaufsatz den Nagel zwangsläufig, wodurch er seine ursprüngliche Länge nicht mehr besitzt.

Es ist von diesem Stand der Technik ausgehend Aufgabe der vorliegenden Erfindung, einen modularen Marknagel anzugeben, der die oben beschriebenen Nachteile nicht aufweist und somit eine grössere biomechanische Stabilität aufweist und eine bessere Vaskularisierung gewährleistet. Ein solcher Marknagel ist im kennzeichnenden Teil von Patentanspruch 1 definiert.

Die Erfindung wird im folgenden anhand einer Zeichnung eines Auführungsbeispiels näher erläutert.
- Figur 1: zeigt in einer Zusammenstellung und teilweisem Schnitt die verschiedenen Elemente des Marknagels,
- Figur 2: zeigt einen Schnitt gemäss II-II in Figur 1,
- Figur 3: zeigt einen Schnitt gemäss III-III in Figur 1,
- Figur 4: zeigt eine Ausschnittsvergrösserung des distalen Nagelendes, und die
- Figuren 5 - 8: zeigen verschiedene Anwendungsmöglichkeiten des erfindungsgemässen Marknagels.

Der Nagel 1 ist im wesentlichen als zylindrischer Stab ausgebildet und kann in ein proximales Teil 2, Mittelteil 3 und distales Teil 4 unterteilt werden. Der in der Zeichnung für den Femur zu verwendende Nagel ist der Anatomie des Femurs angepasst und ist daher in einer sog. Antekurvation gekrümmt. Das distale Teil sowie das Mittelteil sind derart dimensioniert, dass der Nagel unaufgebohrt über einen Führungsdraht in den Markkanal eingebracht werden kann.

Das proximale Teil 2 ist zylindrisch und weist eine Längsbohrung 5 auf, die teilweise mit einem Innengewinde 6 versehen ist, dessen Kerndurchmesser leicht grösser als der Durchmesser der Längsbohrung 5 ist. Diese Bohrung 5 dient der Aufnahme eines Einsatzes 7, dessen Funktion weiter unten erklärt wird. Das proximale Ende 11 des proximalen Teils weist zwei Führungsnuten 12 auf, die der drehsicheren Führung des nicht eingezeichneten Einschlagbügels dienen, auf welchen je nach Indikationsstellung das entsprechende Zielgerät winkelstabil befestigt werden kann. Daher fehlt an diesem Ende der Längsbohrung 5 das Innengewinde, damit es zwischen den Nuten und der Zielgerät-Befestigungs-Schraube nicht zu einem Anfressen kommt.

Das proximale Teil 2 weist ferner einen parallel zur Längsachse verlaufenden, durchgehenden Schlitz 8 auf. In der Längsbohrung 5 befindet sich, quer dazu, ein Führungs-Stift 9, der der Drehsicherung und Positionierung des Einsatzes 7 dient. Unterhalb der Längsbohrung 5, die sich distal über die Längserstreckung des Schlitzes 8 hinaus erstreckt, an seinem distalen Ende, weist das proximale Teil eine Querbohrung 10 auf.

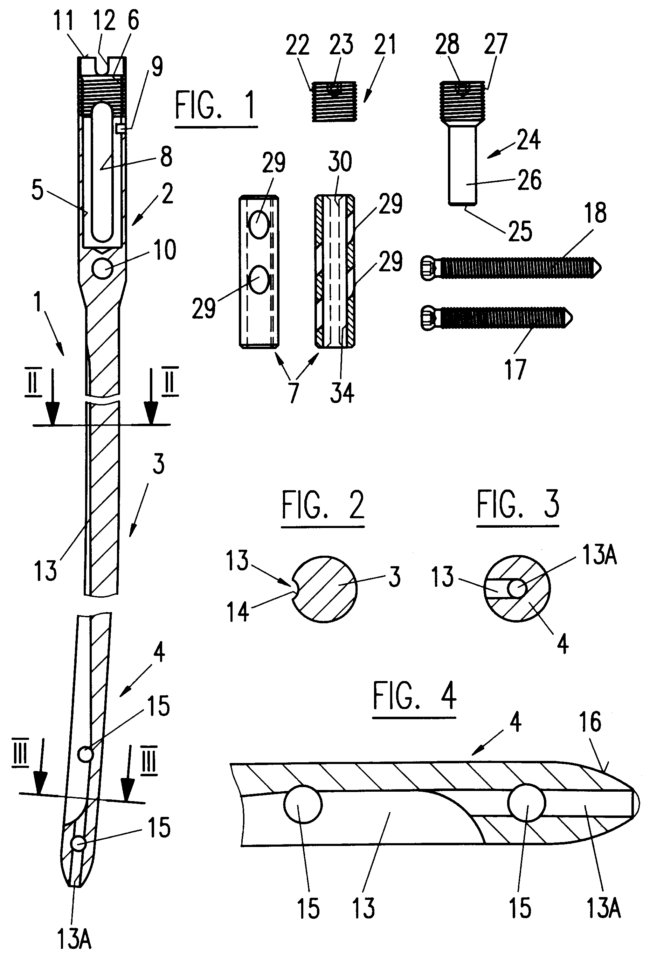

Das Mittelteil 3 und das distale Teil 4 des Nagels haben einen kleineren Durchmesser als das zylinderförmige proximale Teil 2. Aus den Schnitten II-II und III-III geht hervor, dass das Mittelteil an seinem proximalen Ende eine Drahtnut 13 zur Aufnahme eines Führungsdrahtes aufweist, wobei sich die Drahtnut vom distalen Teil nach proximal hinzieht und zur Nagellängsachse geneigt ist. Dies hat zur Folge, dass die Drahtnut unterhalb des proximalen Teils 4 aus dem Nagel austritt, wie dies aus den Figuren 3 und 4 hervorgeht, wobei aus der Drahtnut 13 ein Drahtkanal 13A wird. Der Grund 14 der Nut (siehe Figur 2) ist gerundet, um den Führungsdraht optimal zu führen und eventuell auftretende Kerbspannungen im Grund der Drahtnut zu vermeiden. Diese Art der Führung erlaubt ein sicheres und kontrolliertes Einbringen des Nagels über den Führungsdraht, wobei im Gegensatz zu einer Kanulierung auf nichtvaskularisierten Totraum verzichtet wird.

Wie bereits weiter oben erwähnt weist das distale Teil 4 die Drahtnut 13 sowie den Drahtkanal 13A auf, wobei daraus klar hervorgeht, dass die Ausgestaltung der Führungsmittel für den Führungsdraht dazu führt, dass nicht der ganze Nagel kanuliert ausgeführt werden muss, und dass der durch die Drahtnut reduzierte Nagelquerschnitt die Gefahr einer Fettembolie während dem Einbringen des Nagels reduziert.

Es ist jedoch für die Ausführung der Erfindung nicht unbedingt notwendig, das Mittel- und distale Teil mit einer Drahtnut und einem Drahtkanal zu versehen, eine zentrale Kanulierung des Marknagels ist auch möglich.

Im unteren Abschnitt weist das distale Teil Querbohrungen 15 auf. Die Nagelspitze 16 ist parabolisch ausgebildet, um ohne grossen Widerstand über die Frakturlinie geführt werden zu können. Die mindestens zwei Querbohrungen 15 dienen der Aufnahme der distalen Verriegelungsschrauben 17. Gegebenenfalls kann noch ein Längsschlitz, nicht eingezeichnet, am distalen Teil 4 angebracht sein.

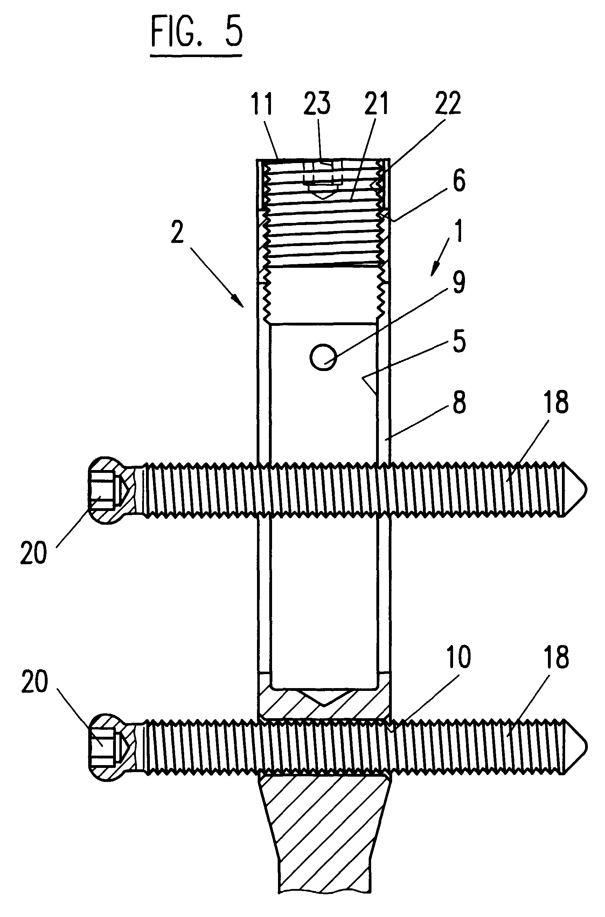

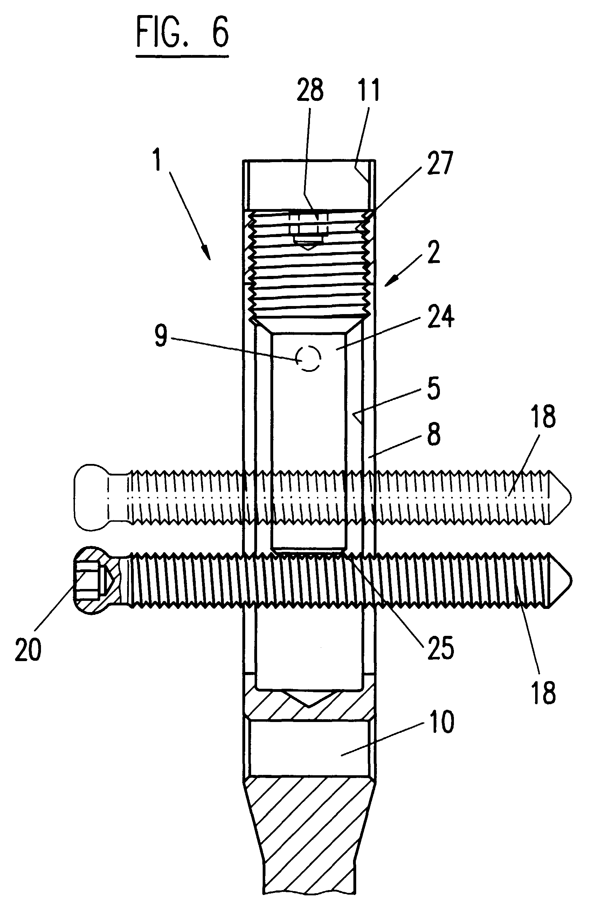

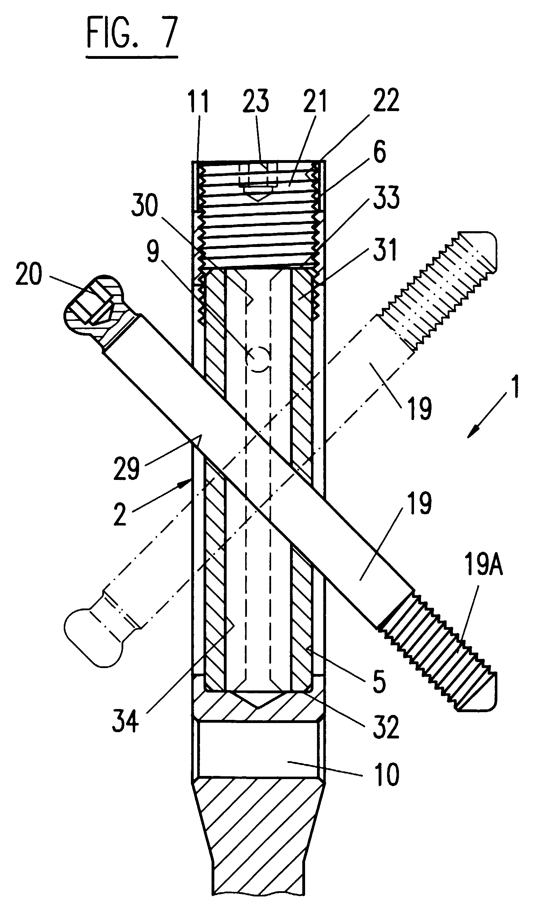

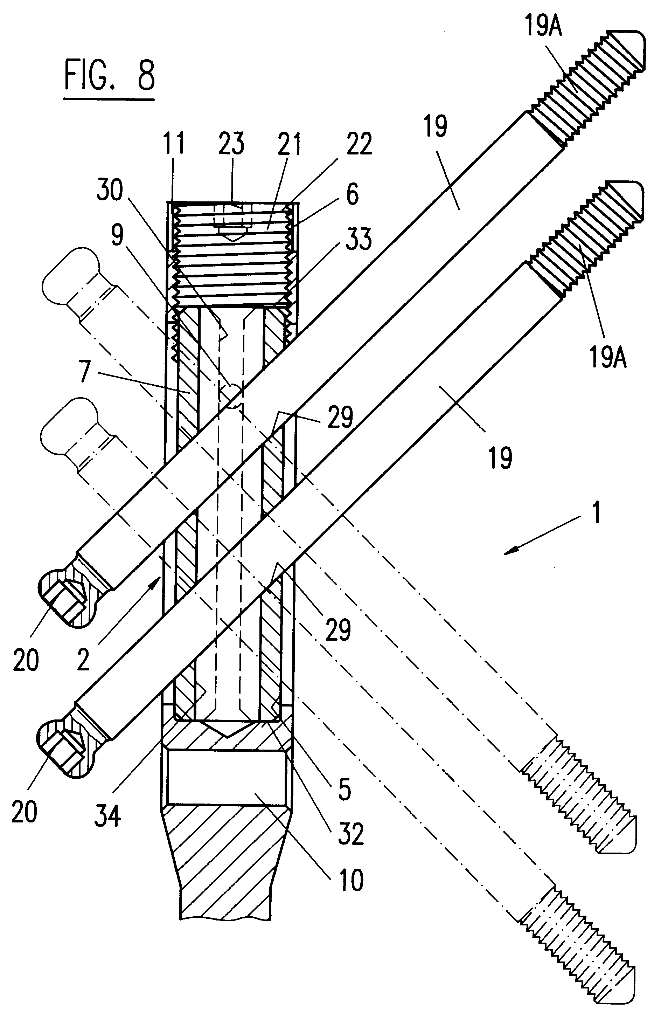

Zur Versorgung von Frakturen im mittleren Schaftbereich kann der Marknagel gemäss den Figuren 5 und 6 eingesetzt werden, wobei die Axial- und die Torsionskräfte mit der Verriegelungsschraube 18 im Querloch 10 sowie durch eine zweite Verriegelungsschraube 18 im Schlitz 8, die nur Torsionskräfte aufnimmt, aufgenommen werden. Statt Verriegelungsschrauben 18 können auch Verriegelungsbolzen 19 (siehe Figuren 7 und 8) verwendet werden, die einen Innensechskant 20 aufweisen.

Die Längsbohrung 5 ist durch eine Verschlussschraube 21 gegen das Einwachsen von Gewebe in den Gewindeteil geschützt, das die Befestigung des Explantations-Instrumentes erschweren würde. Die Verschlussschraube 21 ist mit einem Aussengewinde 22 versehen und weist ein Innensechskant 23 auf, über den die Schraube festgezogen werden kann.

Bei der Versorgung einer Mittschaftfraktur kann mit Hilfe einer Kompressionsschraube 24 (siehe Figuren 6 oder 1) eine aktive Kompressionsverriegelung durchgeführt werden. Dazu wird mit dem proximalen Zielgerät (nicht eingezeichnet) die Verriegelungsschraube 18 quer zur Nagellängsachse in den Längsschlitz 8 eingeführt. Anschliessend wird die Kompressionsschraube 24 im proximalen Gewinde 6 des proximalen Teils eingebracht, bis sie mit ihrem distalen Ende 25 auf der Verriegelungsschraube 18 zu liegen kommt. Indem man nun die Kompressionsschraube weiter eindreht, verschiebt sich das proximale Fragment des Knochens relativ zum Nagel bis sich die Fraktur geschlossen hat und interfragmentäre Kompression aufgetreten ist. Die Kompressionsschraube 24 enthält des weiteren einen Schaftteil 26, dem sich ein Aussengewindeteil 27 mit grösserem Durchmesser anschliesst, sowie einen Innensechskant 28.

Zur wesentlichen Erweiterung des Anwendungsbereichs des erfindungsgemässen Marknagels, insbesondere zur antegraden oder retrograden Verriegelung ist ein Einsatz 7 vorgesehen, der sowohl hohl als auch voll ausgeführt sein kann. Der Einsatz 7 gemäss vorliegendem Ausführungsbeispiel weist eine durchgehende Längsbohrung 34 und zwei bezüglich der Längsachse geneigte Führungsbohrungen 29 auf, wobei es auch mehr als zwei Führungsbohrungen, aber mindestens eine Führungsbohrung, sein können. Dabei entspricht der Aussendurchmesser der Verschlussschraube 21 in etwa dem Aussendurchmesser des Einsatzes 7, damit dieser festgezogen werden kann. Dadurch kann der Einsatz in der Längsbohrung nicht mehr verrutschen, d. h. dass eine genau definierte Position des Einsatzes im Bezug auf die Längsachse vorliegt. Im weiteren kann es durch die Festklemmung zu keinen Mikrobewegungen zwischen der Bohrungsinnenwand und dem Einsatz kommen, die eventuell zu unerwünschtem Abrieb führen könnten. Schliesslich erhöht der Einsatz durch Versteifung die biomechanische Stabilität des Nagels.

Die zur Längsachse geneigten Führungsbohrungen 29 können verschiedene Durchmesser aufweisen und dienen der Aufnahme von Verriegelungsschrauben 18 oder Verriegelungsbolzen 19. Der Einsatz weist ferner eine Führungsnut 30 auf, in die der Führungsstift 9 greift und somit das richtige Einführen des Einsatzes sichert, derart, dass die Führungsbohrungen 29 für die Verriegelungsschrauben bezüglich des Schlitzes 8 des Nagels ausgerichtet sind. Um das Einführen zu erleichtern, ist die Führungsnut an ihren beiden Enden trichterförmig erweitert. Die Führungsbohrungen 29 sind bei einer retrograden Verriegelung der Fraktur vorzugsweise zwischen 120° bis 150° bezüglich der Längsachse ausgerichtet, während bei einer antegraden Verriegelung diese vorzugsweise zwischen 125° und 150° bezüglich der Längsachse ausgerichtet sind.

Figur 7 zeigt die retrograde, bzw. antegrade Verriegelung einer Fraktur mit einem Einsatz 31, der nur eine Führungs-Bohrung 29 aufweist, aber sonst identisch mit dem Einsatz 7 ist. Man erkennt in dieser Figur 7 und auch in Figur 8, wie der Führungsstift 9 in die Führungsnut 30 des Einsatzes eingreift und den Einsatz dadurch genau bezüglich des Längsschlitzes 8 ausrichtet. Es ist ferner ersichtlich, dass durch Einbringen der Hülse mit dem einen Ende 32 in distaler Richtung oder mit dem anderen Ende 33 in distaler Richtung entweder retrograd oder antegrad verriegelt werden kann. In diesem Falle wie im Falle gemäss Figur 8 dient die Verschlussschraube 21 einerseits zum Festklemmen des Einsatzes und ausserdem zum Schutz gegen das Einwachsen von Gewebe in den Gewindeteil.

In Figur 8 ist die Verwendung des Einsatzes 7 mit Zwei Führungsbohrungen 29 bei einer retrograden, bzw. antegraden Verriegelung der Fraktur dargestellt. Sinngemäss gelten die gleichen Ueberlegungen wie zum Einsatz 31 gemäss Figur 7.

Aus obiger Beschreibung geht hervor, dass mit einem Nagel 1 sowie einer beschränkten Anzahl von Einsätzen sämtliche Femurschaftfrakturen sowie die verschiedensten proximalen Femurfrakturen versorgt werden können. Ausserdem können die Einsätze und damit auch die den Einsatz aufnehmende Längsbohrung 5 unabhängig vom Durchmesser oder der Länge des Nagels gestaltet sein. Im weiteren bedarf es keiner linken und rechten Ausführungsform des Nagels, was den Einsatz und die Lagerhaltung dieses Implantates wesentlich begünstigt. Lediglich durch das um 180° gedrehte Einführen des Einsatzes wird die entsprechende Ausführungsform des Nagels links oder rechts festgelegt. Es geht ferner aus der Beschreibung hervor, dass auch andere Ausführungsformen des Marknagels, beispielsweise als Rekonstruktionsnagel, im Sinne der Erfindung verwendet werden können.

## Patentansprüche

1. Marknagel, insbesondere modularer, unaufgebohrter Femur-Marknagel, mit einem aus einem proximalen Teil (2), Mittelteil (3) und distalen Teil (4) bestehenden Nagel (1), wobei das proximale Teil (2) einen Längsschlitz (8) sowie darunter angeordnet mindestens eine Querbohrung (10) und das distale Teil (4) mindestens eine Querbohrung (15) aufweisen, dadurch gekennzeichnet, dass das proximale Teil (2) eine distal sich über die Längserstreckung des Schlitzes (8) erstreckende Längsbohrung (5) und einen Einsatz (7, 31) zum Einsetzen in diese Längsbohrung aufweist, wobei der Einsatz (7 bzw. 31) mindestens eine geneigt bezüglich der Längsachse ausgerichtet verlaufende Führungsbohrung (29) zur Aufnahme von Verriegelungs-Schrauben (18), -Bolzen (19) oder Klingen aufweist.

2. Marknagel nach Anspruch 1, dadurch gekennzeichnet, dass der Einsatz (7, 31) zur retrograden Verriegelung mit seinem einen Ende (32) und zur antegraden Verriegelung mit seinem anderen Ende (33) in distaler Richtung in der Längsbohrung (5) einsetzbar und verriegelbar ist.

3. Marknagel nach Anspruch 1 oder 2, dadurch gekennzeichnet, dass in die Längsbohrung (5) im proximalen Teil (2) ein Führungsstift (9) hineinragt und der Einsatz (7, 31) mit einer entsprechenden Führungsnut (30) zum lagerichtigen Einsetzen versehen ist.

4. Marknagel nach einem der Ansprüche 1 bis 3, dadurch gekennzeichnet, dass die Längsbohrung (5) im proximale Teil (2) mit einem Innengewinde (6) versehen ist und eine Verschlussschraube (21) aufweist.

5. Marknagel nach einem der Ansprüche 1 bis 4, dadurch gekennzeichnet, dass er eine in das Innengewinde (6) der Längsbohrung (5) im proximale Teil (2) schraubbare Kompressionsschraube (24) aufweist.

6. Marknagel nach einem der Ansprüche 1 bis 5, dadurch gekennzeichnet, dass der Einsatz (7) hohl oder voll ist.

7. Marknagel nach einem der Ansprüche 1 bis 6, dadurch gekennzeichnet, dass das Mittelteil (3) und das distale Teil (4) des Nagels (1) mit einer Drahtnut (13) versehen ist, die im distalen Ende in einen Drahtkanal (13A) mündet.

8. Marknagel nach Anspruch 7, dadurch gekennzeichnet, dass der Nutgrund (14) der Drahtnut (13, 13A) gerundet ist.
